# EUROPEAN PATENT APPLICATION

(11) **EP 1 588 763 A2**
(43) Date of publication of application: **26.10.2005**
(21) Application number: 05008062.1
(22) Date of filing: 13.04.2005
(51) Int. Cl.: B01J 37/02, B01J 23/58, B01J 23/60, B01J 23/66, C07C 67/055

(54) **Production process for catalyst for producing vinyl acetate**

(30) Priority: 14.04.2004 JP 2004118478
(71) Applicant: N.E. Chemcat Corporation, Minato-ku, Tokyo (JP)
(72) Inventor: Inoue, Atsushi c/o Numazu Jigyosho, Numazu-shi Shizuoka-ken (JP); Yamamoto, Hiroshi c/o Numazu Jigyosho, Numazu-shi Shizuoka-ken (JP)
(74) Representative: Tönhardt, Marion

(57) **Abstract**

A production process for a catalyst that supports a noble metal, and an alkali metal compound and/or alkali earth metal compound, useful for synthesis of vinyl acetate is provided. The process includes (a) impregnating a carrier with an aqueous solution of a water soluble noble metal compound, and (b) converting the noble metal compound into a water insoluble noble metal compound, and the noble metal compound is reduced, followed by supporting an alkali metal compound and/or alkali earth metal compound. An element that oxidizes to produce an amphoteric oxide is brought into contact with the carrier at the step (a) and/or the step (b). This process is advantageous in facilities required and the associated running costs for production of an eggshell-type structure catalyst, which offers a high level of catalytic activity, and enables the production of vinyl acetate with a high space-time yield.

## Description

### BACKGROUND OF THE INVENTION

### 1. Field of the Invention

The present invention relates to a production process for a catalyst, and more particularly to a production process for a catalyst that is useful in a vinyl acetate synthesis that enables vinyl acetate to be produced with a high space-time yield, using ethylene, acetic acid, and oxygen as raw materials.

### 2. Description of the Prior Art

Conventionally, syntheses of vinyl acetate that are conducted in the presence of palladium-gold-alkali metal compound-supporting silica catalysts are well known. Furthermore, although early catalysts for vinyl acetate synthesis contained the active components distributed uniformly throughout the entirety of the carrier particles, including the particle interior, subsequent research revealed quite clearly that the synthesis reaction of vinyl acetate occurs entirely at the catalyst surface, meaning the active components within the interior of the carrier particles were not contributing to the reaction. Accordingly, it is now widely known that supporting the active components on the surface layer of the carrier particles enables a more efficient synthesis of vinyl acetate (see patent reference 1). Supporting the noble metal that functions as the principal active component on the surface layer of the carrier particles is particularly desirable.

In recent years, so-called eggshell catalysts, in which the active component is supported only on the surface layer of the carrier, have become mainstream. In a production process for an eggshell catalyst, a carrier is impregnated uniformly with an aqueous solution of a water soluble noble metal compound, and by subsequently contacting and reacting this impregnated carrier with an aqueous solution of a basic compound, the water soluble noble metal compound is converted to a water insoluble noble metal compound, which is removed out towards the carrier surface, and supported in the surface layer of the carrier.

Subsequently, the water insoluble noble metal compound is reduced to form the noble metal in an elementary state, and the material is washed and dried, yielding a noble metal-supporting carrier. An alkali metal compound and/or alkali earth metal compound is then supported on this noble metal-supporting carrier, completing preparation of an eggshell catalyst.

However, in the reduction step of the water insoluble noble metal compound in the above production process, if a liquid phase reduction process using hydrazine or the like is employed, then because large quantities of the active component are supported on carrier particles with smaller surface areas, the distance between active component particles tends to shrink, causing aggregation of the active component particles, which leads to an increase in the particle size, and a particularly large increase in the size of the noble metal particles in an elementary state. This causes a decrease in the noble metal specific surface area, meaning even though the majority of the active component is supported on the surface layer, an improvement in the catalytic activity that corresponds with this increase in the quantity of the active component is not observed.

Based on the above problems, a gas phase reduction process has been proposed (see patent reference 2), which does not require the above liquid phase reduction step, but instead uses a gaseous reducing agent such as hydrogen gas. In this gas phase reduction process, the water insoluble noble metal compound component supported on the surface layer of the carrier is able to be reduced to the noble metal with no increase in the particle size. Accordingly, a catalyst with a high catalytic activity can be produced in which the noble metal is supported on the carrier surface layer, and the noble metal particle size is small, that is, the associated specific surface area is large.

However, employing this gas phase reduction process requires large scale facilities, and because the gas phase reduction step is conducted in a non-continuous manner, during a production process that is conducted primarily in the liquid phase, the production process is more complicated and additionally the running costs associated with the production of the catalyst are very high.
[Patent Reference 1] U.S. Pat. No. 3,939,199
[Patent Reference 2] U.S. Pat. No. 6,303,536 B1

As described above, production of an eggshell catalyst useful for vinyl acetate synthesis using a gas phase reduction process suffers the drawback of requiring large scale facilities.

### SUMMARY OF THE INVENTION

Accordingly, a principal object of the present invention is to provide a catalyst for vinyl acetate synthesis, which is produced without the need for a gas phase reduction process, using a liquid phase reduction process that is advantageous in terms of the facilities required and the associated running costs, is of an eggshell-type structure, displays a noble metal specific surface area that matches or exceeds that of a catalyst produced by a gas phase reduction process, offers a high level of catalytic activity, and enables the production of vinyl acetate with a high space-time yield.

As a result of intensive investigations aimed at achieving the above object, the inventors of the present invention discovered that, prior to the liquid phase reduction step, by incorporating an element that oxidizes to produce an amphoteric oxide within at least one of the water soluble noble metal compound that is impregnated into the carrier, and the aqueous solution of a basic compound that is used to treat the impregnated carrier, a catalyst can be obtained in which the particle size of the noble metal particles following reduction remains small, and the noble metal surface area is large, and they were hence able to complete the present invention.

In other words, the present invention provides a production process for a catalyst that supports a noble metal, and an alkali metal compound and/or alkali earth metal compound, comprising the following steps of:
(a) impregnating a carrier with an aqueous solution of a water soluble noble metal compound,
(b) bringing the impregnated carrier into contact with an aqueous solution of a basic compound, thereby converting the noble metal compound into a water insoluble noble metal compound,
(c) reducing the water insoluble noble metal compound, and generating an elementary noble metal-supporting carrier in which the noble metal is supported in a surface layer of the carrier, and
(d) supporting an alkali metal compound and/or alkali earth metal compound on said noble metal-supporting carrier,
said process comprising bringing an element that oxidizes to produce an amphoteric oxide in a state of aqueous solution into contact with said carrier at at least one of said step (a) and said step (b).

The present invention also provides a catalyst produced by this production process, and a production process for vinyl acetate that is conducted in the presence of the catalyst.

According to the production process of the present invention, a catalyst with a high level of catalytic activity can be produced, in which the noble metal surface area following liquid phase reduction has been increased to a value that is at least as large as the palladium specific surface area of a catalyst obtained by a gas phase reduction. Conducting a gas phase reaction of ethylene, acetic acid, and oxygen in the presence of this catalyst enables the synthesis of vinyl acetate with a high space-time yield.

### DETAILED DESCRIPTION OF THE PREFERRED EMBODIMENTS

As follows is a more detailed description of the present invention.

### [Step (a)]

The carrier used in the present invention can utilize any inert carrier without any particular restrictions, and suitable examples include alumina, silica, and activated carbon, although silica is particularly preferred.

The primary active component of a catalyst produced using the production process of the present invention (hereafter also referred to as a "catalyst of the present invention") is a noble metal, examples of which include palladium, gold, silver, platinum, rhodium, and ruthenium. These noble metals may be used singly or in combination of two or more thereof. Particularly, the combination of palladium and gold is the most desirable.

A conventional method is used to support the noble metal component onto the aforementioned carrier, by using a water soluble noble metal compound such as an aqueous solution ofNa₂[PdCl₄], H₂[PdCl₄], K₂[PdCl₄], H[AuCl₄], K[AuCl₄], Na[AuCl₄], or K[Au(CN)₂], and absorbing and impregnating this solution into a carrier such as silica. In those cases where some of water soluble noble metal compounds are used, either the above absorption and impregnation can be conducted using a mixed aqueous solution of the various compounds, thereby supporting the compounds onto the carrier in a simultaneous manner, or individual aqueous solutions of each of the water soluble noble metal compounds may be used in a sequential manner.

### [Step (b)]

Subsequently, the impregnated carrier is brought into contact with an aqueous solution of a basic compound. Specifically, a device is used wherein either the impregnated carrier is immersed in an aqueous solution of a basic compound, or the aqueous solution of the basic compound is either dripped or poured onto the impregnated carrier.

In this step, the reaction with the basic compound converts the aforementioned water soluble noble metal compound into a water insoluble noble metal compound. During this process, the noble metal compound is simultaneously removed to the surface layer of the carrier, thereby supporting the water insoluble noble metal compound on the carrier surface layer.

Examples of the basic compound include hydroxides and other salts of alkali metals and alkali earth metals, and specific examples include NaOH, KOH, Na₂[SiO₃]·9H₂O, Na₂CO₃, and NaHCO₃.

In a preferred embodiment of the process of the present invention, in order to suppress the aggregation of the principal active component during the liquid phase reduction step described below, at least one of the aqueous solution of the water soluble noble metal compound used in the aforementioned step (a), and the aqueous solution of the basic compound used in the aforementioned step (b) contains an element that oxidizes to produce an amphoteric oxide, and this represents a characteristic feature of the present invention.

Specific examples of the aforementioned element that oxidizes to produce an amphoteric oxide include Zn, Cd, Al, Ga, In, Si, Sn, Pb, As, Sb, and Bi. Of these, the elements Zn and Al, which do not poison the noble metal catalyst component, are particularly preferred. These elements are used, for example, in the form of water soluble inorganic compounds. Examples of compounds of these elements include salts such as chlorides and nitrates. In addition, specific examples of the compounds include ZnCl₂, AlCl₃·6H₂O, Zn(NO₃)₂, and Al(NO₃)₃.

In the above step (a), this compound of the above element may be mixed with the aqueous solution of the water soluble noble metal compound, and the resulting mixed solution brought into contact with and impregnated into the carrier, or alternatively, an aqueous solution of the compound of the element may be prepared and brought into contact with and impregnated into the carrier separately from the aqueous solution of the water soluble noble metal compound.

Furthermore, in the alternative, in the above step (b), the compound of the above element may be mixed with the aqueous solution of the basic compound, or alternatively, an aqueous solution of the compound of the element may be brought into contact with and impregnated into the carrier separately from the aqueous solution of the basic compound. Moreover, the compound of the above element may also be mixed and used in both the step (a) and the step (b), in the manner described above.

### [Steps (c) to (d)]

After the above steps (a) and (b), production is continued in the same manner as a conventional production process for a catalyst for vinyl acetate synthesis using a liquid phase reduction. Namely, the aforementioned water insoluble noble metal compound is reduced using a reducing agent such as, e.g., hydrazine, sodium borohydride, alcohol, or formic acid, thus forming the elementary noble metal (i.e., simple noble metal) such as palladium or gold, and the carrier is then washed with pure water and dried, yielding a noble metal-supporting carrier.

An aqueous solution of an alkali metal compound and/or alkali earth metal compound is then impregnated uniformly into the above noble metal-supporting carrier and dried, thus yielding a catalyst of the present invention wherein the alkali metal compound and/or alkali earth metal compound is supported on the noble metal-supporting carrier.

Examples of the above alkali metal compound and/or alkali earth metal compound include the hydroxides, nitrates, acetates, and carbonates of sodium, potassium, cesium, magnesium, calcium, and barium, and of these, potassium acetate is particularly preferred.

### [Quantities of Supported Active Components]

The quantity of the noble metal, which represents the primary active component, supported in a catalyst of the present invention is typically within a range from 0.1 to 10.0% by mass, and preferably from 0.1 to 3.0% by mass in total relative to the total mass of the catalyst of the present invention. When two or more noble metals are supported in the catalyst, the quantities of each of the noble metals supported in a catalyst of the present invention are typically within a range from 0.1 to 10.0% by mass, and preferably from 0.1 to 3.0% by mass, for each noble metal, relative to the total mass of the catalyst of the present invention. For example, in the case of a combination of palladium and gold, the supported quantities of palladium and gold are typically each within a range from 0.1 to 10.0% by mass, and preferably from 0.1 to 3.0% by mass, relative to the total mass of the catalyst of the present invention.

Furthermore, the supported quantity of the alkali metal compound and/or alkali earth metal compound such as potassium acetate is typically within a range from 1 to 15% by mass, and preferably from 3 to 10% by mass, relative to the mass of the catalyst of the present invention.

The quantity used of the compound of the element that produces an amphoteric oxide, which as described above, is used for suppressing the aggregation of particles of the principal active component, is typically within a range from 0.01 to 5% by mass, and preferably from 0.1 to 1.0% by mass, relative to the mass of the catalyst of the present invention.

In the present invention, by using a water soluble compound of an element that oxidizes to produce an amphoteric oxide, aggregation of the elementary noble metal particles generated by the liquid phase reduction can be suppressed. This suppressing effect is markedly exhibited for, particularly, palladium. It is surmised that the reason for this effect is that during the liquid phase reduction of the aforementioned step (c), the existence of the water soluble compound of the above element within the system inhibits the movement of the noble metal particles generated by the reduction reaction, thereby suppressing aggregation of the noble metal particles.

### [Method of Measuring Noble Metal Surface Area]

The surface area of the noble metal particles can be measured by chemically adsorbing carbon monoxide to the noble metal particles supported on the surface layer of the carrier, and then calculating the noble metal surface area from the quantity of adsorbed carbon monoxide.

For example, a prescribed quantity of the catalyst to be measured is weighed and packed into a measurement cell. The cell is then placed inside a sealed container, and a pretreatment is conducted at a constant temperature of 40°C by passing helium gas, hydrogen gas, and then helium gas through the container, in that sequence. Subsequently, under the same temperature conditions, a fixed quantity of carbon monoxide is injected into the container at intermittent intervals, until the adsorbed quantity reaches an equilibrium.

The total quantity (volume) of adsorbed carbon monoxide is deemed the saturated adsorption quantity, and is used to determine the number of molecules of adsorbed carbon monoxide for conditions of 40°C and atmospheric pressure. The surface area of a noble metal particle occupied by each molecule of adsorbed carbon monoxide varies depending on the crystalline structure of the noble metal particle, but in the case of palladium, is 8.24 × 10⁻²⁰ m². The noble metal specific surface area (m²/g) is calculated based on the value for this occupied surface area, and the total number of molecules of adsorbed carbon monoxide.

### [Method of Measuring Space-time Yield]

Space-time yield STY, which represents the evaluation standard for catalytic activity in the synthesis of vinyl acetate, can be calculated by filling a reaction vessel with the catalyst, supplying and reacting ethylene, oxygen, and acetic acid to synthesize vinyl acetate, and determining the mass of generated vinyl acetate per unit of time, and per unit of catalyst volume (g/L-cat/hr).

This vinyl acetate synthesis reaction is conducted at a reaction temperature (catalyst temperature) of 100 to 200°C, and preferably from 130 to 180°C, and at a reaction pressure from normal pressure to 3 MPa, and preferably from normal pressure to 1 MPa. Furthermore, the gas supplied to the reaction system is a mixed gas comprising ethylene, oxygen, and acetic acid, and the gas used for evaluating the reactivity typically comprises from 70 to 90 vol% of ethylene, from 5 to 10 vol% of oxygen, and from 5 to 20 vol% of (gaseous) acetic acid (wherein the combination of these three gases is 100 vol%), relative to the total volume of supplied gas.

If required, the reactivity can also be evaluated with nitrogen, carbon dioxide, or water vapor or the like added to the gas.

Furthermore, the alkali metal compound and/or alkali earth metal compound such as potassium acetate supported on the catalyst is known to gradually desorb from the carrier during the reaction, and exit from the reaction system. In order to maintain the catalytic activity, an aqueous solution or acetic acid solution of potassium acetate or the like can be added to the supply gas, thus enabling the alkali metal compound and/or alkali earth metal compound such as potassium acetate to be replenished within the catalyst in the reaction system.

### EXAMPLES

As follows is a more detailed description of the present invention based on a series of examples and comparative examples, although the present invention is in no way limited to the examples presented below. As described below, in this description, an example in which a catalyst is produced using a conventional liquid phase reduction process is termed comparative example 1, and an example in which a catalyst is produced using a gas phase reduction process is termed comparative example 2, and these comparative examples are presented before the examples.

### [Comparative Example 1]

In order to support 3.5 g of metallic Pd and 1.5 g of metallic gold on 1 L of a silica carrier (KA-160, manufactured by Süd Chemie Group), aqueous solutions of Na₂[PdCl₄] and H[AuCl₄] were weighed and combined, and were then diluted with pure water to meet the saturated adsorption quantity for the silica carrier, which had been determined in advance. The dilute solution was absorbed into 1 L of the carrier, thus supporting the above Pd compound and Au compound on the carrier.

33.7 g of Na₂[SiO₃]·9H₂O was dissolved in pure water, and the solution volume was made up to 430 mL. The silica carrier supporting the above Pd compound and Au compound was placed in this aqueous solution, and left immersed in the solution for at least 16 hours at room temperature.

29 g of an aqueous solution of N₂H₄·H₂O with a concentration of 80% by weight was then added to the above aqueous solution, and the solution was left to stand for 4 hours to effect a reduction treatment of the Pd compound and the Au compound. Following this reduction treatment, the Pd and Au-supporting silica carrier was removed, and washed with pure water. Washing was continued until the wash liquid displayed no turbidity on the dropwise addition of a silver nitrate solution, and the carrier was then dried, yielding a Pd and Au-supporting silica carrier.

Subsequently, 30 g of an aqueous solution of potassium acetate with a concentration of 60% by weight was diluted with pure water to produce a volume equivalent to the saturated absorption quantity of the Pd and Au-supporting silica carrier. This dilute solution was then absorbed into the Pd and Au-supporting silica carrier, and following supporting of the potassium acetate, the carrier was dried, thus completing preparation of the catalyst.

The noble metal (Pd) specific surface area of the thus obtained catalyst was 154 (m²/g-Pd).

This catalyst was used to fill a reaction vessel, and a vinyl acetate synthesis was then conducted by supplying a mixed gas of ethylene: oxygen: acetic acid (volumetric ratio) = 83:7:10 to the reaction vessel, under conditions including a reaction temperature (catalyst temperature) of 140°C and a reaction pressure of 0.78 MPa. The space-time yield of vinyl acetate was 398 (g/L-cat/hr).

### [Comparative Example 2]

In the same manner as the comparative example 1 described above, a Pd compound and a Au compound were supported on a silica carrier, and this silica carrier supporting the Pd compound and the Au compound was then immersed in an aqueous solution of Na₂[SiO₃]·9H₂O.

Following this immersion treatment, the silica carrier supporting the Pd compound and the Au compound was removed from the solution and washed with pure water. Washing was continued until the wash liquid displayed no turbidity on the dropwise addition of a silver nitrate solution, and the carrier was then dried.

The thus obtained silica carrier supporting the Pd compound and the Au compound was then placed in a hydrogen reduction furnace, and subjected to gas phase reduction under a stream of hydrogen at a temperature of 300°C.

Potassium acetate was then supported on the carrier in the same manner as the comparative example 1, thus yielding a catalyst.

The noble metal (Pd) specific surface area of the thus obtained catalyst was 257 (m²/g-Pd).

When this catalyst was used in a vinyl acetate synthesis under the same conditions as the comparative example 1, the space-time yield of vinyl acetate was 591 (g/L-cat/hr).

### [Example 1]

With the exception of adding 0.6 g of ZnCl₂ to the mixed solution generated during the weighing and mixing of the Na₂[PdCl₄] aqueous solution and the H[AuCl₄] aqueous solution, a catalyst was prepared in the same manner as the comparative example 1.

The noble metal (Pd) specific surface area of the thus obtained catalyst was 253 (m²/g-Pd).

When this catalyst was used in a vinyl acetate synthesis under the same conditions as the comparative example 1, the space-time yield of vinyl acetate was 623 (g/L-cat/hr).

### [Example 2]

With the exception of replacing the step in the comparative example 1 in which 33.7 g of Na₂[SiO₃]·9H₂O was dissolved in pure water and the solution volume was made up to 430 mL, with a step in which 29.0 g of AlCl₃·6H₂O and 24.0 g of NaOH were dissolved in pure water and the solution volume was made up to 430 mL, a catalyst was prepared in the same manner as the comparative example 1.

The noble metal (Pd) specific surface area of the thus obtained catalyst was 171 (m²/g-Pd).

When this catalyst was used in a vinyl acetate synthesis under the same conditions as the comparative example 1, the space-time yield of vinyl acetate was 440 (g/L-cat/hr).

### [Evaluation]

The space-time yields and the noble metal (Pd) specific surface area values for the catalysts obtained in the comparative example 1, the comparative example 2, the example 1, and the example 2 are shown in Table 1.

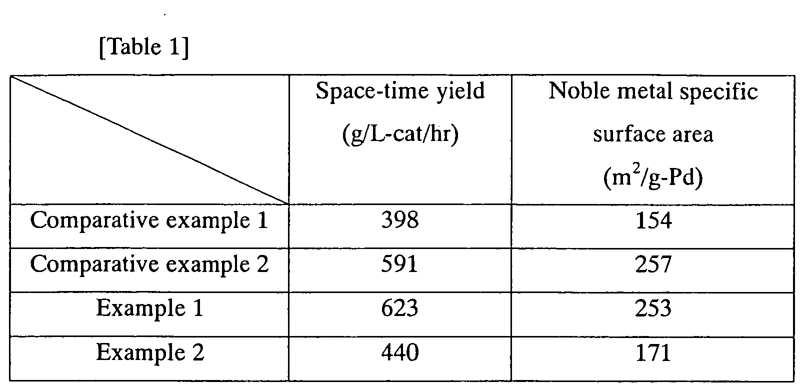

From the above results it can be confirmed that compared with the catalyst produced by a conventional liquid phase reduction process in the comparative example 1, the catalysts of the example 1 and the example 2, which were produced using the process of the present invention, display a larger noble metal (Pd) specific surface area, have a smaller particle size for the noble metal particles, and provide an increased space-time yield and a higher catalytic activity during vinyl acetate synthesis.

Furthermore, it is also evident that compared with the catalyst produced by a gas phase reduction process in the comparative example 2, the catalyst of the example 1 displays similar or slightly superior performance in terms of the noble metal (Pd) surface area and the space-time yield. Furthermore, because the process of the present invention does not require the gas phase reduction facilities that are needed during the production of a catalyst using a gas phase reduction process, it offers considerable production advantages.

The features disclosed in the foregoing description and / or in the claims may, both separately and in any combination thereof, be material for realising the invention in diverse forms thereof.

## Claims

1. A production process for a catalyst that supports a noble metal, and an alkali metal compound and/or alkali earth metal compound, comprising the following steps of:
(a) impregnating a carrier with an aqueous solution of a water soluble noble metal compound,
(b) bringing said impregnated carrier into contact with an aqueous solution of a basic compound, thereby converting said noble metal compound into a water insoluble noble metal compound,
(c) reducing said water insoluble noble metal compound, and generating an elementary noble metal-supporting carrier in which said noble metal is supported in a surface layer of said carrier, and
(d) supporting an alkali metal compound and/or alkali earth metal compound on said noble metal-supporting carrier,
said process comprising bringing an element that oxidizes to produce an amphoteric oxide in a state of aqueous solution into contact with said carrier at at least one of said step (a) and said step (b).

2. The production process according to claim 1, wherein at least one of the aqueous solution of a water soluble noble metal compound used at said step (a) and said aqueous solution of a basic compound used at said step (b) contains said element that oxidizes to produce an amphoteric oxide.

3. The production process according to claim 1, wherein said noble metal comprises at least one member of palladium, gold, silver, platinum, rhodium, and ruthenium.

4. The production process according to claim 1, wherein said noble metal is a combination of palladium and gold.

5. The production process according to claim 1, wherein said alkali metal compound and/or alkali earth metal compound is potassium acetate.

6. A catalyst produced by the production process according to claim 1

7. A production process for vinyl acetate, which comprises reacting ethylene, acetic acid, and oxygen in presence of a catalyst according to claim 6 in a gas phase.
